# EUROPEAN PATENT SPECIFICATION

(11) **EP 1 778 860 B1**
(45) Date of publication and mention of the grant of the patent: **16.01.2008**
(21) Application number: 05781699.3
(22) Date of filing: 28.07.2005
(51) Int. Cl.: C12Q 1/533, G01N 33/573, G01N 33/88

(54) **METHODS FOR DETERMINING THE POTENCY, SPECIFICITY, AND TOXICITY OF HEMATOPOIETIC PROSTAGLANDIN D2 SYNTHASE**
VERFAHREN ZUR BESTIMMUNG DER WIRKSAMKEIT, DER SPEZIFITÄT UND DER GIFTIGKEIT DER BLUTBILDENDEN PROSTAGLANDIN-D2-SYNTHASE
METHODES POUR DETERMINER LA PUISSANCE, LA SPECIFICITE ET LA TOXICITE DE LA PROSTAGLANDINE D2 SYNTHASE HEMATOPOIETIQUE

(30) Priority: 30.07.2004 US 592501 P
(43) Date of publication of application: 02.05.2007
(73) Proprietor: Aventis Pharmaceuticals Inc., Bridgewater, New Jersey 08807-0800 (US)
(72) Inventor: CAO, Yang, Bridgewater, NJ 08807 (US); SABOL, Jeffrey, S., Bridgewater, New Jersey 8807 (US); AYERS, Stephen, Swedesboro, NJ 08085 (US)
(74) Representative: Bouvet, Philippe
(86) International application number: PCT/US2005/026945
(87) International publication number: WO 2006/015195

(56) References cited:
- CA-A1- 2 503 674
- US-A1- 2004 082 021
- KANAOKA Y ET AL: "HEMATOPOIETIC PROSTAGLANDIN D SYNTHASE" PROSTAGLANDINS LEUKOTRIENES AND ESSENTIAL FATTY ACIDS, CHURCHILL LIVINGSTONE MEDICAL JOURNALS,, GB, vol. 69, no. 2/3, 2003, pages 163-167, XP009019772 ISSN: 0952-3278
- CIPOLLONE F ET AL: "Balance between PGD synthase and PGE synthase is a major determinant of atherosclerotic plaque instability in humans" ARTERIOSCLEROSIS, THROMBOSIS, AND VASCULAR BIOLOGY 2004 UNITED STATES, vol. 24, no. 7, 20 May 2004 (2004-05-20), pages 1259-1265, XP002351016 ISSN: 1079-5642
- MURAKAMI YOUSUKE ET AL: "Inhibition of monosodium urate monohydrate crystal-induced acute inflammation by retrovirally transfected prostaglandin D synthase." ARTHRITIS AND RHEUMATISM. OCT 2003, vol. 48, no. 10, October 2003 (2003-10), pages 2931-2941, XP002351017 ISSN: 0004-3591

## Description

### FIELD OF THE INVENTION

The present invention relates to a novel and useful method for assaying compounds and agents for their ability to decrease or inhibit the activity of hematopoietic prostaglandin D2 synthase (hPGDS). Specifically, the present invention relates to assays for measuring the potency, specificity and toxicity of hPGDS inhibitors.

### BACKGROUND OF THE INVENTION

Prostaglandins are a class of eicosanoids that have diverse biological effects. Prostaglandins have been shown to have pharmacological effects on the cardiovascular system, platelet aggregation, effects on smooth muscle thereby effecting the gastrointestinal system, kidney function and urine formation, effects on the central nervous system, endocrine system, effects on metabolism and play a critical role in inflammation and immune responses. Thus, prostaglandins represent the potential to have broad therapeutic utility. Prostaglandins are synthesized from arachidonic acid, and possess a five-membered ring of carbon atoms that had formed part of the chain of arachidonic acid. Typically, prostaglandins act locally, i.e., near the site of their synthesis.

Prostaglandin D2 (PGD2) is the major prostanoid produced by activated mast cells in response to antigen challenge, which is a major mediator of airway allergic disorders. In particular, it causes, among other things, bronchoconstriction, bronchial hyper-responsiveness, nasal congestion and eosinophil and TH2 cell infiltration. It is also thought that released PGD2 from antigen-stimulated mast cells and basophils elicit immediate hypersensitivity, airway inflammation or remodeling in asthma.

The enzyme prostaglandin D synthase (PGDS) catalyzes the conversion of prostaglandin H2 (PGH2) into PGD2. Two types of PGD2 synthases are known. The first is a lipocalin-type (L-PGDS) found mainly in the central nervous system, and the second is hematopoietic PGDS (hPGDS), which is found primarily in peripheral tissues. L-PGDS is glutathione (GSH)-independent while hPGDS is GSH-dependent. Furthermore, there is very little structural homology between L-PGDS and hPGDS.

Since PGD2 plays an important role in inflammation, efforts have been made to develop assays to screen for compounds that may inhibit their production. In particular, inhibitors of PGD2 synthesis may be valuable in the treatment of allergic rhinitis, asthma and possibly chronic obstructive pulmonary disease (COPD). The therapeutic application may also be extended to the treatment of multiple sclerosis, Alzheimer's disease and disorders resulting from ischemia-reperfusion injury, brain tumor and therapeutic applications in biological processes modulated by prostaglandins.

Techniques such as high pressure liquid chromatography (HPLC), enzyme-linked immunosorbent assays (ELISA), also known as enzyme immunoassays (EIA), or radioimmunoassays (RIA) are used to quantify the production of PGD2 in order to determine a compound's or agent's ability to decrease, inhibit, enhance or modulate the production of PGD2.

In the past, assessments of potency, specificity and toxicity have been separated into different experimental paradigms. Moreover, specificity determinations were more feasible in biochemical assays that in many cases could not mimic the complex biological conditions existing in cells. Thus, it is desirable to design a cell based assay that measures potency, specificity and toxicity of PGDS modulators from the same experimental sample. Such a design would shorten the steps that usually require secondary screening and provide information that is more closely relevant to the cell conditions in vivo.

### BRIEF SUMMARY OF THE INVENTION

The present invention establishes a single cell-based assay to evaluate potency, specificity, and cytotoxicity simultaneously for hPGDS modulators. Compounds that modulate the activity of hPGDS provide novel therapeutic approaches for the treatment of inflammation and pharmacological manipulation of immune responses. The assay design is suitable for use in any mammalian cell line in which hPGD2 is expressed such as mast cells, antigen-presenting cells, megakaryocytes and Th2 lymphocytes. In the present invention, the mammalian cells are treated with a stimulatory molecule that enhances arachidonic acid release. Molecules contemplated in the present invention are calcium ionophores such as A23187, ionomycin, phorbel esters, growth factors, cytokines, tumor promoters, or a calcium channel mobilizer molecules such as BAY K-8644. In one particular embodiment, arachidonic acid may be directly added to the cells.

Cells used to screen for compounds that modulate hPGDS are treated with the test compound and a stimulatory molecule to induce arachidonic acid release. PGD2 production levels are measured in the presence of test compound and compared with PGD2 levels without test compound pre-treatment. In addition to measuring the potency of a test compound, the present invention provides for specificity and cytotoxicity measurements all in the same cell-based assay system. This is designed by evaluating a second readout for PGE2. For example, if a test compound specifically inhibits hPGDS activity, it will generate an IC50 on PGD2 that is significantly lower than that on PGE2. In contrast, if the test compound is non-specific or cytotoxic, it will generate similar IC50 values on PGD2 and PGE2. Additional cytotoxicity testing can be used to determine whether these values are due to the toxicity of the test compound or non-specific inhibition of prostanoid synthesis.

The above aspects and other aspects, features, and advantages of the present invention will be better understood from the following detailed description taken in conjunction with the accompanying figures.

### BRIEF DESCRIPTION OF THE DRAWINGS

FIG 1 shows the results of the cell-based assay distinguishing between specific and non-specific PGD2 inhibitors. Compound A000050350 specifically inhibits PGD2 production in RBL cells whereas compound A000127348 non-specifically inhibits both PGD2 and PGE2 production in RBL cells.
FIG 2 is a graph representing the evaluation of cytotoxicity by measuring cell proliferation of rat basophilic leukemia (RBL) cells treated with several concentrations of PGDS test compounds using the CellTiter 96 Aqueous One Solution Cell Proliferation Assay.

### DETAILED DESCRIPTION OF THE INVENTION

The present invention relates to a cell-based assay for screening compounds that modulate hematopoietic PGD2 synthase (hPGDS). The assay evaluates potency, specificity and toxicity of test compounds in living cells. The present invention allows for the identification of inhibitor compounds that may be used as drugs to treat many inflammatory diseases including but not limited to asthma, allergic rhinitis, COPD, multiple sclerosis, Alzheimer's disease and other disorders resulting from ischemia-reperfusion injury.

The present invention generally relates to a cell based assay, which is used to measure potency, specificity and cytotoxicity of test compounds that modulate hPGDS activity within a single cell based assay. Measurements can be performed quickly and can be scaled up to test many different compounds simultaneously.

There are no particular restrictions as to the compound that can be assayed. Examples include small molecular weight chemical molecules, libraries of synthetic low molecular compounds, purified proteins, expression products of gene libraries, synthetic peptide libraries, cell extracts and culture supernatants.

Any mammalian cell line may be used in the present invention as long as the mammalian cell line expresses hPGDS. Examples include, but are not limited to, mast cells, antigen-presenting cells, megakaryocytes and Th2 lymphocytes. In addition, mammalian cell lines may be used such as rat basophilic leukemia (RBL). Primary mast cells in different species such as human and mouse, or T cell subtypes in different species are the additional examples. Cells engineered to express or overexpress hPGDS or modified hPGDS are contemplated by the present invention.

The chosen mammalian cells are treated with a stimulatory molecule that enhances arachidonic acid release. Molecules contemplated for use in the present invention are calcium ionophores such as A23187, ionomycin, phorbel esters, growth factors, cytokines, tumor promoters, and calcium channel mobilizer molecules such as BAY K-8644. In another particular embodiment of the present invention, arachidonic acid may be directly added to the chosen mammalian cells.

To measure potency in the present invention, cells are treated with a test compound followed by the addition of a stimulatory molecule to enhance arachidonic acid release. Levels of PGD2 production are measured in cells treated with the stimulatory molecule and various concentrations of test compounds. These levels are compared to the levels of PGD2 produced in cells that were treated with stimuli but were not exposed to test compound.

The present invention also provides in the same cell-based assay the ability to measure specificity and cytotoxicity. This is designed by evaluating a second readout of prostanoid production, for example, levels of PGE2, in the cell supernatant. If a test compound specifically inhibits hPGDS activity, it will generate an IC50 on PGD2 that is significantly lower than that on PGE2. In contrast, if the test compound is non-specific or cytotoxic, it will generate similar IC50 values for both PGD2 and PGE2. To determine whether the non-specific inhibition is due to toxicity of the test compound, an additional cytotoxicity assay may be set up subsequently using the same samples as in the cell-based assay, or separately using the same conditions as the cell-based assay described above. A finding of inhibition of cell proliferation is considered a measurement of off-target activity of the tested compound.

Cytotoxicity assays that may be used in the present invention include but are not limited to assays such as MTS, MTT and LDH assays, all commercially available from Promega, for example.

The ability of a compound to modulate levels of PGD2 is determined, not just by taking measurements in the presence and absence of the inhibitor compound, but also at various concentrations thereof. By taking measurements at various compound concentrations, it is possible to calculate certain features, such as its IC50. An IC50 is defined as the concentration at which the compound produces 50% inhibition. Conversely, the assay of the present invention is easily amendable to assay for compounds that stimulate PGD2 production by using a sub-threshold amount of stimulatory molecule or no stimulatory molecule.

It is also understood that the assay of the present invention may also be conducted as a single point assay evaluating the inhibition of compound on PGD2 and PGE2 production using only one concentration of test compound. Both single point screening and IC50 determinations are amenable to high throughput screening. High throughput screening systems are commercially available (see, e.g., Zymark Corp., Hopkington, Mass.; Air Technical Industries, Mentor, Ohio; Beckman Instruments, Inc. Fullerton, Calif.; Precision Systems, Inc., Natick, Mass., etc.). These systems typically automate entire procedures including all sample and reagent pipetting, liquid dispensing, timed incubations, and final reading of the samples in detector(s) appropriate for the assay. These configurable systems provide high throughput and rapid start up as well as a high degree of flexibility and customization. The manufacturers of such systems provide detailed protocols for the various high throughput assays.

Methods of measuring concentrations of PGD2 and PGE2 are well known in the art. Examples include high pressure liquid chromatography (HPLC), gas chromatography mass spectrometry (GC-MS), enzyme-linked immunosorbent assay (ELISA), also known as enzyme immunoassay (EIA), radioimmunoassay (RIAs), and fluorescence polarization (FP). The scintillation proximity assay may also be useful in measuring PGD2 and PGE2 levels.

The present invention is not limited in scope by the specific embodiments described herein. Indeed, various modifications of the invention in addition to those described herein will become apparent to those skilled in the art from the foregoing description and the accompanying figures. Such modifications are intended to fall within the scope of the appended claims.

### EXAMPLES

### EXAMPLE 1: CELL TREATMENT WITH CALCIUM IONOPHORE AND INCUBATION WITH TEST AND CONTROL COMPOUNDS

In this example, RBL-2H3 cells (obtained from the American Tissue Culture Center) are trypsinized (0.05% trypsin EDTA in prepared MEM media (Gibco 11095-080) supplemented with 10% fetal bovine serum, L-glutamine, penicillin and streptomycin to a single-cell suspension. RBL cells are plated at 2x10⁴ cells/well in 96 well plates. The plates are incubated overnight at 37°C.

HBSS buffer (Gibco/Invitrogen 14025-092) is diluted with DMSO (Sigma D-8779) equivalent to the highest concentration of DMSO contained in the reference or test compound. The compound plate(s) and reference control are thawed and mixed well before use. In this example, a COX-1 inhibitor SC560 was used as a reference and purchased from Cayman Chemical.

Test compound and reference SC560 dilutions are made with a Costar (3960 or equivalent) assay block according to the EIA template shown below. Column 1 wells (Blank, TA=Total activity, NSB=non-specific binding and Bo=background) and columns 2 & 3 are not used for cell treatments, but are filled with standards for the EIA assay. Controls 1, 2, 5 and 6 are positive controls containing calcium ionophore without compound. Controls 3, 4, 7 and 8 are negative controls without calcium ionophore or compound.

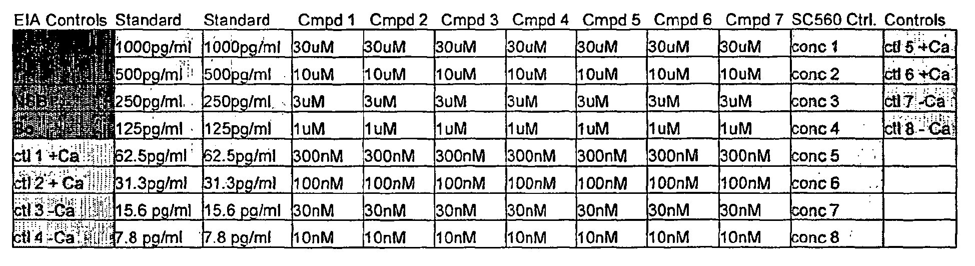

Test compounds were diluted as follows: Add 1 ml of HBSS (without DMSO) to assay block wells A4-A10 [30 uM]. Add 620 ul of HBSS w/ 0.3% DMSO to wells B4-B10 through H4-H10. Add 3 ul of compound stock [10 mM] to 30 uM wells. Mix well. Add 310 ul from row A [30 uM] to row B [10 uM]. Mix well. Continue diluting as above, to generate the following concentrations [30, 10, 3.3, 1.1, 0.37, 0.12, 0.04, and 0.014 uM].

The reference SC560 was diluted as follows: Add 1 ml of HBSS (without DMSO) to well A11. Add 620 ul of HBSS with 0.3% DMSO to wells B11-H11. Add 3 ul of pre-diluted stock [1 mM in 100% DMSO] to well A11 [3 uM]. Mix well. Add 310 ul from well A11 [3 uM] to well B11 [1 uM]. Mix well. Continue diluting to generate the following concentrations [3 uM, 1 uM, 0.33 uM, 0.11 uM, 0.037 uM, 0.012 uM, 0.0041 uM, and 0.0013 uM].

An automated cell treatment for the addition of calcium ionophore (A23187 Sigma #C-7522) and methoximating reagent (prepared according to Cayman kit #512011 directions) was designed using a Zymark SciClone Deck.

The present automated (A) protocol requires that a number of steps be performed manually (M) as outlined below.

### (A) Remove overnight media and wash 1x with 200 ul HBSS buffer.

Remove HBSS buffer from cells and replace with 200 ul/well diluted compounds and controls according to template. (M) Cover and return cell plates to 37 °C. Incubate for 15 minutes. Uncover cell plates and return to deck. (A) Add 10 ul of 20x concentration to 200 ul cell treatments including positive control wells. Note there is no addition of calcium ionophore to negative control wells. (M) Remove empty tip boxes and replace with 200 ul tips at deck locations B2, B3, and B4. (M) Cover and return cell plates to 37 °C. Incubate for 15 minutes. (M) Centrifuge plates @ 1000 x rpm for 5 minutes. Uncover cell plates and return to deck. (A) Collect 25 ul supernatants to be chemically methoximated with 25ul MOX reagent (1:1 ratio) according to PGD2-MOX EIA kit (Cayman #512011 or equivalent). See following example. (M) Cover MOX assay plates and incubate @ 60 °C for 30 minutes. (M) Place additional assay plates at deck locations D2, D3, and D4 for supernatants to be collected for PGE2 analysis. (A) Collect 175 ul of remaining supernatant to be evaluated for PGE2. Methoximated (for PGD2) and un-methoximated (for PGE2) supernatants can be stored for up to 3 months @ -80 °C.

### EXAMPLE 2: QUANTIFICATION OF PROSTAGLANDIN D2 LEVELS: ENZYME IMMUNOASSAY

In this example, the concentration of PGD2 was measured using an enzyme immunoassay (EIA). EIAs for measuring PGD2 are available commercially. For example a PGD2 EIA kits are available from Cayman Chemical.

This assay is based on the competition between PGD2-methoxime (MOX) and a PGD2-MOX-acetylcholinesterase (AchE) conjugate (PGD2-MOX tracer) for a limited number of PGD2-MOX-specific rabbit antiserum binding sites. Because the concentration of the PGD2-MOX tracer is held constant while the concentration of PGD2-MOX varies, the amount of PGD2-MOX tracer that is able to bind to the rabbit antiserum will be inversely proportional to the concentration of PGD2-MOX in the well. This rabbit antiserum-PGD2-MOX complex binds to the mouse monoclonal anti-rabbit IgG that has been previously attached to the well. After washing the plate, Ellman's Reagent (which contains the substrate to AchE) is added to the well. The enzymatic product has a distinct yellow color, which absorbs strongly at 412 nm. The intensity of this color is proportional to the amount of PGD2-MOX tracer bound to the well, which is inversely proportional to the amount of free PGD2-MOX present in the well during the incubation.

The EIA and wash buffers were made according to the commercial instructions. Samples were prepared according to the previous example. Since prostaglandin levels may vary, the supernatants are diluted with EIA buffer to yield prostaglandin levels within the linear range of the standard curve. Commonly used PGD2 dilutions are 1:12.5.

The methyl oximating reagent, PGD2-MOX Express EIA standard, PGD2-MOX AchE Express Tracer, PGD2-MOX Express Antiserum and PGD2-MOX Express EIA Methoximating control were prepared according to the manufacturer's instructions. The assay was performed according to the manufacturer's instructions and the plates were read at a wavelength of 412 nm.

After the prostaglandin levels were determined in cells treated with 8 concentrations of test compounds, an IC50 of the compound may be generated by conducting a semi-log plot of the prostaglandin level versus log scale of the compound concentration. This assay can also be conducted as a single point assay evaluating the inhibition of compound on PGD2 production using only one concentration of compound.

### EXAMPLE 3: QUANTIFICATION OF PROSTAGLANDIN E2 LEVELS

In this example, the PGE2 concentrations were measured using an enzyme immunoassay (EIA). EIAs for measuring PGE2 are available commercially. For example a PGE2 EIA kit is available from Assay Designs. The PGE2 EIA employs a mouse monoclonal antibody specific for PGE2. The free PGE2 in solution competes with a known amount of PGE2 tracer to bind a limited amount of the anti-PGE2 antibody. The PGE2 tracer is a conjugate of PGE2 and acetylcholinesterase. The anti-PGE2 antibody is then fixed using a goat anti-mouse Ig antibody. The fixed antibody is then developed with Ellman's Reagent, which contains the substrate for acetylcholinesterase. The product produced by this reaction has a yellow color, and absorbs strongly at 412 nm. If there is a high concentration of PGE2, the PGE2 will outcompete the tracer and the resulting sample will have a weak absorbance at 412 nm. Conversely, if there is a low concentration of PGE2, the tracer will outcompete the PGE2 and the resulting sample will have a strong absorbance at 412 nm. In performing this assay, the absorbance at 412 nm was compared to a standard of absorbances at known concentrations of PGE2.

This assay may be easily converted to automated format by one skilled in the art by using a Zymark SciClone Deck, for example.

### EXAMPLE 4: QUANTIFICATON OF PGD2 AND PGE2 to distinguish SPECIFIC AND NON-SPECIFIC COMPOUNDS

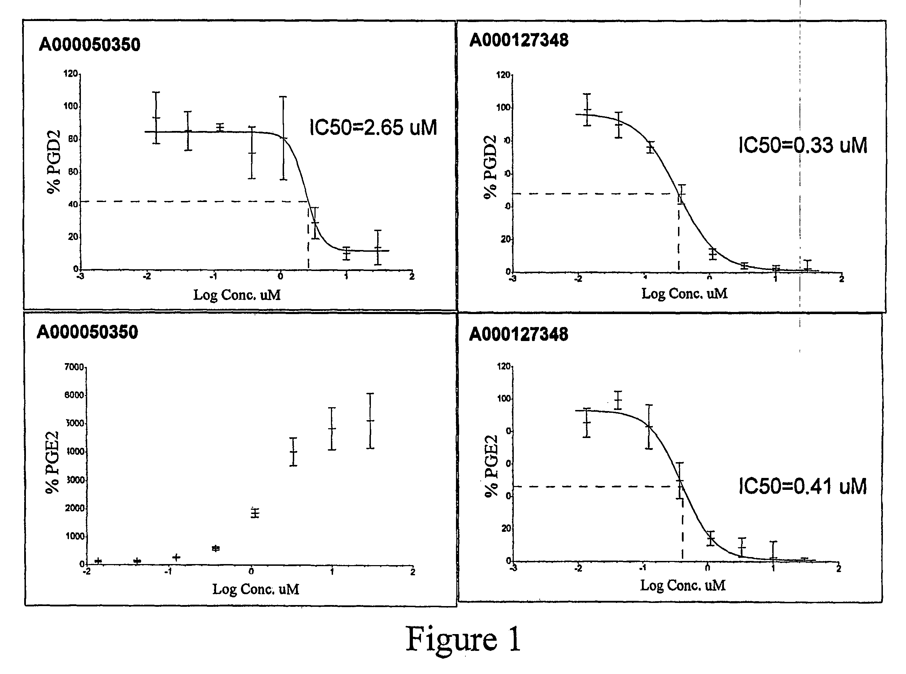

This example shows how the above PGD2 and PGE2 cell-based assays may be used to distinguish between specific and non-specific compounds. As shown in Figure 1, compound A00050350 generates an IC50 value of 2.65uM relating to PGD2 inhibition, but no inhibition of PGE2 was exhibited, suggesting that A0050350 is a potent and specific inhibitor on PGD2 synthesis. In contrast, compound A000127348 generates similar IC50 values for PGD2 and PGE2; 0.33 uM and 0.41 uM, respectively, suggesting that A000127348 is a non-specific or cytotoxic compound. A subsequent cytotoxicity study confirmed that compound A000127348 affects cell proliferation under the testing conditions.

In this example, RBL cells were treated with the PGDS compounds for 45 min under the same condition as in the cell-based assay except no A23187 was added. The potential cytotoxicity was determined with the CellTiter 96 Aqueous One Solution Cell Proliferation Assay (MTS) (Promega, Madison, WI Cat# G3582). The assay was carried out following the manufacturer's instruction.

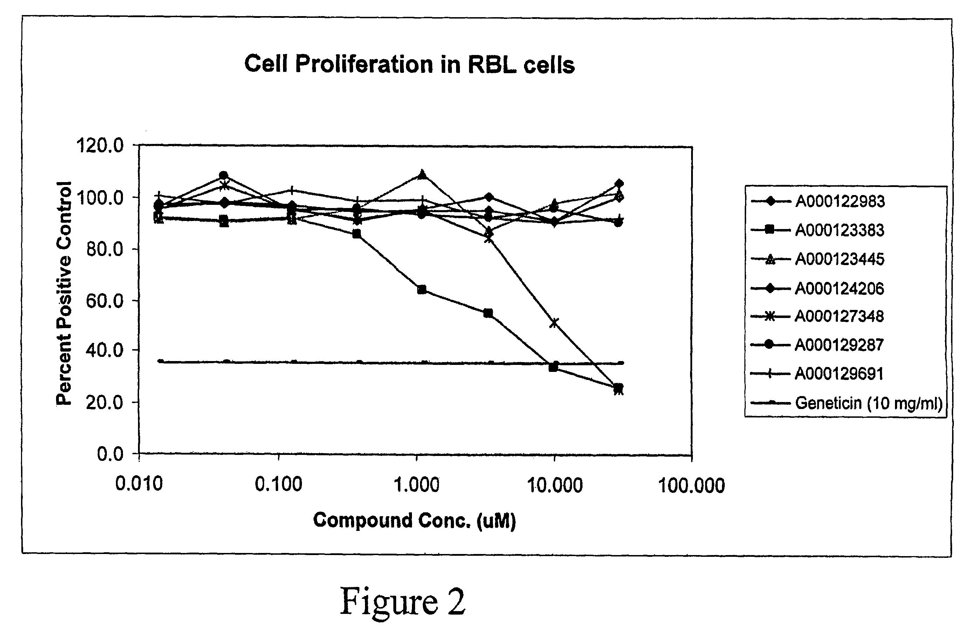

Eight concentrations of each compound ranging from 0.01uM to 30uM were determined for cytotoxicity. The testing concentrations were 0.014uM, 0.041uM, 0.123uM, 0.370uM, 1.111uM, 3.333uM, 10uM and 30uM (See Figure 2). Compound A000127348 and another PGDS compound, A000123383, were toxic, which are distinguishable from a number of non-toxic test compounds shown in the same plot. Geneticin was used as a positive control.

## Claims

1. An in vitro method for screening for potency, specificity and toxicity of inhibitor compounds of hematopoietic prostaglandin D₂ synthase (hPGDS) in mammalian cells comprising the steps of:
i) contacting said mammalian cell with a stimulatory molecule that increases the release of arachidonic acid,
ii) treating said mammalian cell with a compound,
iii) measuring the level of prostaglandin D₂ (PGD2) in said mammalian cell or the supernatant,
iv) comparing the levels of PGD2 in said mammalian cell to the levels of PGD2 in a control mammalian cell that is not treated with said compound to determine the potency of said compound,
v) measuring the level of prostaglandin E2 (PGE2) in said mammalian cell
vi) comparing the levels of PGD2 with the levels of PGE2 in said mammalian cell wherein finding the levels of PGD2 is lower than the levels of PGE2 indicate the compound's specific inhibitory activity of hPGDS,
vii) measuring the amount of cytotoxicity of compound treated and control mammalian cells and comparing the cytotoxicity levels in treated and control mammalian cells wherein a finding of cytotoxicity in compound treated cells is an indication of inhibition of cell proliferation.

2. The method of claim 1 wherein said mammalian cell is selected from the group consisting of mast cells, antigen-presenting cells, megakaryocytes and TH2 lymphocytes.

3. The method of claim 1 wherein said mammalian cell is a rat basophilic leukemia (RBL).

4. The method of claim 1 wherein said stimulatory molecule is selected from the group consisting of calcium ionophores, ionomycin, phorbel esters, growth factors, cytokines, tumor promoters and calcium channel mobilizer molecules.

5. The method of claim 4 wherein said calcium ionophore is A23187.

6. The method of claim 4 wherein said calcium channel mobilizer molecule is BAY K-8644.

7. The method of claim 1 wherein said stimulatory molecule is arachidonic acid.

## Patentansprüche

1. In-vitro-Verfahren für die Untersuchung der Wirksamkeit, Spezifität und Toxizität von Hemmstoffen gegen die hämatopoetische Prostaglandin-D2-Synthase (hPGDS) in Säugetierzellen, bei dem man:
i) die Säuretierzellen mit einem stimulierenden Molekül, das die Freisetzung von Arachidonsäure erhöht, in Kontakt bringt,
ii) die Säugetierzelle mit einer Verbindung behandelt,
iii) die Konzentration von Prostaglandin D2 (PGD2) in der Säugetierzelle oder im Überstand mißt,
iv) die Konzentrationen von PGD2 in der Säugetierzelle mit den Konzentrationen von PGD2 in einer nicht mit der Verbindung behandelten Kontrollsäugetierzelle vergleicht, um die Wirksamkeit der Verbindung zu bestimmen,
v) die Konzentration von Prostaglandin E2 (PGE2) in der Säugetierzelle mißt,
vi) Die Konzentrationen von PGD2 mit den Konzentrationen von PGE2 in der Säugetierzelle vergleicht, wobei der Befund, daß die Konzentrationen an PGD2 niedriger sind als die Konzentrationen an PGE2, darauf hindeutet, daß die Verbindung eine hPGDS spezifisch hemmende Wirkung hat,
vii) das Ausmaß der Zytotoxizität der mit der Verbindung behandelten Säugetierzellen und der Kontrollsäugetierzellen mißt und das Ausmaß der Zytotoxizität in den behandelten Säugetierzellen und den Kontrollsäugetierzellen vergleicht, wobei der Befund einer Zytotoxizität in mit Verbindung behandelten Zellen ein Hinweis auf eine Inhibierung der Zellproliferation ist.

2. Verfahren nach Anspruch 1, wobei die Säugetierzelle ausgewählt ist aus der Gruppe bestehend aus Mastzellen, antigenpräsentierenden Zellen, Megakaryozyten und TH2-Lymphozyten.

3. Verfahren nach Anspruch 1, wobei es sich bei der Säugetierzelle um eine basophile Leukämiezelle der Ratte (rat basophilic leukemia, RBL) handelt.

4. Verfahren nach Anspruch 1, wobei das stimulierende Molekül ausgewählt ist aus der Gruppe bestehend aus/ Calciumionophoren, Ionomycin, Phorbolestern, Wachstumsfaktoren, Zytokinen, Tumorpromotoren und calciumkanalmobilisierenden Molekülen.

5. Verfahren nach Anspruch 4, wobei es sich bei dem Calciumionophor um A23187 handelt.

6. Verfahren nach Anspruch 4, wobei es sich bei dem calciumkanalmobilisierenden Molekül um BAYK-8644 handelt.

7. Verfahren nach Anspruch 1, wobei es sich bei dem stimulierenden Molekül um Arachidonsäure handelt.

## Revendications

1. Procédé in vitro de criblage en ce qui concerne l'efficacité, la spécificité et la toxicité de composés inhibiteurs de la prostaglandine D2 synthase hématopoïétique (hPGDS) dans des cellules de mammifère comprenant les étapes consistant à :
i) mettre en contact ladite cellule de mammifère avec une molécule stimulatrice qui augmente la libération d'acide arachidonique,
ii) traiter ladite cellule de mammifère avec un composé,
iii) mesurer le taux de prostaglandine D2 (PGD2) dans ladite cellule de mammifère ou dans le surnageant,
iv) comparer les taux de PGD2 dans ladite cellule de mammifère avec les taux de PGD2 dans une cellule de mammifère témoin qui n'est pas traitée avec ledit composé, pour déterminer l'efficacité dudit composé,
v) mesurer le taux de prostaglandine E2 (PGE2) dans ladite cellule de mammifère,
vi) comparer les taux de PGD2 avec les taux de PGE2 dans ladite cellule de mammifère, la découverte du fait que les taux de PGD2 sont inférieurs aux taux de PGE2 étant indicatrice de l'activité inhibitrice spécifique du composé vis-à-vis de la hPGDS,
vii) mesurer la quantité de cytotoxicité dans les cellules de mammifère traitées avec le composé et les cellules de mammifère témoins puis comparer les niveaux de cytotoxicité dans les cellules de mammifère traitées et témoins, la découverte d'une cytotoxicité dans les cellules traitées avec le composé étant indicatrice de l'inhibition de la prolifération cellulaire.

2. Procédé selon la revendication 1, dans lequel ladite cellule de mammifère est choisie dans le groupe constitué de mastocytes, de cellules de présentation de l'antigène, de mégacaryocytes et de lymphocytes TH2.

3. Procédé selon la revendication 1, dans lequel ladite cellule de mammifère est de leucémie basophile de rat (RBL).

4. Procédé selon la revendication 1, dans lequel ladite molécule stimulatrice est choisie dans le groupe constitué d'ionophores calciques, d'ionomycine, d'esters de phorbol, de facteurs de croissance, de cytokines, de promoteurs tumoraux et de molécules mobilisant les canaux à calcium.

5. Procédé selon la revendication 4, dans lequel ledit ionophore calcique est A23187.

6. Procédé selon la revendication 4, dans lequel ladite molécule mobilisant les canaux à calcium est BAY K-8644.

7. Procédé selon la revendication 1, dans lequel ladite molécule stimulatrice est l'acide arachidonique.
